# EUROPEAN PATENT SPECIFICATION

(11) **EP 4 347 612 B1**
(45) Date of publication and mention of the grant of the patent: **15.07.2026**
(21) Application number: 22735717.5
(22) Date of filing: 01.06.2022
(51) Int. Cl.: C07F 17/00, C07C 13/44, C07C 49/633

(54) **METHODS OF MAKING ALKYLATED METALLOCENES HAVING ONE OR MORE TETRAHYDROPENTALENYL GROUPS**
VERFAHREN ZUR HERSTELLUNG VON ALKYLIERTEN METALLOCENEN MIT EINER ODER MEHREREN TETRAHYDROPENTALENYLGRUPPEN
PROCÉDÉS DE FABRICATION DE MÉTALLOCÈNES ALKYLÉS AYANT UN OU PLUSIEURS GROUPES TÉTRAHYDROPENTALÉNYLE

(30) Priority: 02.06.2021 US 202163195797 P
(43) Date of publication of application: 10.04.2024
(73) Proprietor: Dow Global Technologies LLC, Midland, MI 48674 (US)
(72) Inventor: PADILLA-ACEVEDO, Angela, I., Lake Jackson, Texas 77566 (US); BRENNAN, Tamara, L., Lake Jackson, Texas 77566 (US); DUVVURI, Krishnaja, Midland, MI 48640 (US); OGAWA, Kelli, A., Midland, MI 48640 (US)
(74) Representative: Boult Wade Tennant LLP
(86) International application number: PCT/US2022/031705
(87) International publication number: WO 2022/256363

(56) References cited:
- WO-A1-2019/067272

## Description

### Field of Disclosure

Embodiments of the present disclosure are directed towards methods of making metallocenes, more specifically, making alkylated metallocenes having one or more tetrahydropentalenyl groups.

### Background

Metallocenes can be used in various applications, including as polymerization catalysts, as antiknock additives in gasoline, and as lubricants, as well as potential use in medical applications such as cancer, malaria and bacterial infection treatments.

### Summary

The present disclosure provides various embodiments, including: a method of making an alkylated metallocene having one or more tetrahydropentalenyl groups, the method comprising: reacting a chloride compound with a lithium compound to make a reaction mixture comprising an intermediate dichloride compound; and reacting the intermediate dichloride compound with an alkylating agent to make the alkylated metallocene having one or more tetrahydropentalenyl groups, wherein the dichloride compound is not isolated from the reaction mixture, wherein the lithium compound is represented by: 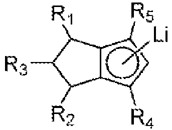 wherein R₁, R₂, R₃, R₄, and R₅ are each independently H or a (C1-C4) alkyl, and wherein the reactions occur in a same reaction vessel.

### Detailed Description

Previously, alkylated metallocenes have been made by reacting a chloride compound, which contains a metal atom, with a lithium compound to form an intermediate compound that includes the metal atom and a number of chlorine atoms. The reaction forming the intermediate compound can be referred to as a metalation reaction. Following the metalation reaction, the intermediate compound is generally isolated from a reaction mixture that contains unused reactants, byproducts, and/or side-products. Then, the isolated intermediate compound is reacted with an alkylating agent to make the alkylated metallocene.

While not wishing to be bound to theory, it is believed that prior methods of making alkylated metallocenes have isolated one or more intermediate compounds to separate the intermediate compounds from byproducts, to change to different solvents for different reaction steps, to separate the intermediate compounds from side-products, and/or reduce the likelihood of low product yields, e.g. from competing reactions. Byproducts are made as a result of a desired reaction. Side-products are made from side reactions. WO 2019/067272 A1 disclose the preparation of cyclic organic compounds and substituted metallocenes.

Surprisingly, it has been found that alkylated metallocenes having one or more tetrahydropentalenyl groups can be made without isolating the intermediate compound from the reaction mixture. In other words, the alkylated metallocenes having one or more tetrahydropentalenyl groups can be made without isolating the intermediate compound from the reaction mixture prior to reacting the intermediate compound and the alkylating agent. Further it has been found that methods of making alkylated metallocenes having one or more tetrahydropentalenyl groups, as disclosed herein, can provide an improved, i.e. greater, product yield percentage, as compared to alkylated metallocenes made with different methods. Further it has been found that methods of making alkylated metallocenes having one or more tetrahydropentalenyl groups, as disclosed herein, can provide an improved, i.e. reduced, amount of byproducts and/or side-products, as compared to other methods of making alkylated metallocenes having one or more tetrahydropentalenyl groups.

As used herein, all reference to the Periodic Table of the Elements and groups thereof is to the NEW NOTATION published in HAWLEY'S CONDENSED CHEMICAL DICTIONARY, Thirteenth Edition, John Wiley & Sons, Inc., (1997) (reproduced there with permission from IUPAC), unless reference is made to the Previous IUPAC form noted with Roman numerals (also appearing in the same), or unless otherwise noted.

Embodiments provide alkylated metallocenes having one or more tetrahydropentalenyl groups can be made by reacting a chloride compound with a lithium compound to make a reaction mixture comprising an intermediate dichloride compound; and reacting the intermediate dichloride compound with an alkylating agent to make the alkylated metallocene having one or more tetrahydropentalenyl groups.

Embodiments provide that the chloride compound can be represented as MClₙ, where M is a metal, Cl is chlorine atoms, and n is an integer from 3 to 4. Embodiments provide that M can be zirconium, hafnium, or titanium. Embodiments provide that the chloride compound includes a plurality of chlorine atoms. For instance, one or more embodiments provide the chloride compound includes 3 or 4 chlorine atoms. One or more embodiments provide the chloride compound is selected from zirconium (IV) chloride and n-(propylcyclopentadienyl)hafniumtrichloride (dimethoxyethane).

Embodiments provide that the lithium compound can be represented by the following formula: 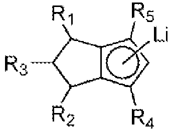 wherein R₁, R₂, R₃, R₄, and R₅ are each independently H or a (C1-C4) alkyl, and wherein the reactions occur in a same reaction vessel. One or more embodiments provide that at least one of R₁, R₂, R₃, R₄, and R₅ are a (C1-C4) alkyl. One or more embodiments provide that at least two of R₁, R₂, R₃, R₄, and R₅ are a (C1-C4) alkyl. One or more embodiments provide that R₁, R₂, R₃ are each H, and R₄, and R₅ are a (C1-C4) alkyl. One or more embodiments provide that R₁, R₂, R₃ are each H, and R₄, and R₅ are a (C1) alkyl.

As mentioned, the chloride compound and the lithium compound are reacted to make a reaction mixture comprising an intermediate dichloride compound. One or more embodiments provide that the intermediate dichloride compound can be represented by the following formula that includes one or more, i.e. two, tetrahydropentalenyl groups: 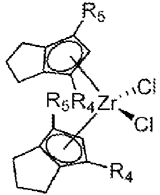 where R₄, and R₅ are as defined above. One or more embodiments provide that the intermediate dichloride compound can be represented by the following formula that includes one tetrahydropentalenyl group:

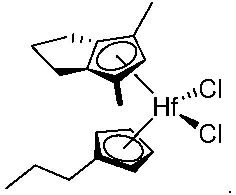

As shown in the above formulas, the intermediate dichloride compound includes two chlorine atoms. Further as shown in the above formulas, the intermediate dichloride compound includes one or more tetrahydropentalenyl groups.

Embodiments provide that the reaction mixture that is made by reacting the chloride compound and the lithium compound can include the intermediate dichloride compound, unreacted chloride compound, unreacted lithium compound, one or more byproducts, one or more side-products, and/or one or more known reaction components, e.g., a solvent. As mentioned, it has advantageously been found that methods of making alkylated metallocenes having one or more tetrahydropentalenyl groups, as disclosed herein, can provide an improved, i.e. reduced, amount of byproducts and/or side-products, as compared to other methods of making alkylated metallocenes having one or more tetrahydropentalenyl groups.

Embodiments further provide that the reaction mixture does not include an alkylating agent. In other words, reacting the chloride compound with the lithium compound to make the reaction mixture comprising the intermediate dichloride compound occurs in absence of an alkylating agent.

Embodiments provide that the intermediate dichloride compound is not isolated from the reaction mixture prior to a subsequent chemical reaction, i.e., an alkylation reaction. In other words, as disclosed herein, making the alkylated metallocenes having one or more tetrahydropentalenyl groups by reacting the chloride compound with the lithium compound to make the reaction mixture comprising the intermediate dichloride compound and reacting the intermediate dichloride compound with an alkylating agent to make the alkylated metallocene having one or more tetrahydropentalenyl groups may be referred to as a one-pot reaction.

Examples of isolation procedures include distillation, extraction, filtration, and decantation. As such, "not isolated" indicates that the intermediate dichloride compound remains with other reagents used and/or generated in the reaction that makes the intermediate dichloride compound prior to a subsequent chemical reaction for the synthesis of the alkylated metallocenes having one or more tetrahydropentalenyl group. In other words, the purity of the intermediate dichloride compound is not increased prior to a subsequent chemical reaction for the synthesis of the alkylated metallocenes having one or more tetrahydropentalenyl group.

Embodiments provide that the intermediate dichloride compound is reacted with an alkylating agent to make the alkylated metallocene having one or more tetrahydropentalenyl groups. In other words, as the intermediate dichloride compound is not isolated from the reaction mixture, i.e., the alkylating agent is added to reaction mixture that includes the intermediate dichloride compound.

Alkylating agents are known in the art and may be obtained commercially. The alkylating agent may be synthesized by a known process, e.g. by utilizing known components and known conditions. One or more embodiments provide that the alkylating agent is a Grignard reagent, e.g., an alkyl Grignard reagent, which may be referred to as an alkylmagnesium halide. Examples of alkylating agents include, methylmagnesium bromide, methylmagnesium chloride, methyllithium, phenylmagnesium bromide, benzylmagnesium bromide, trimethylsilylmethyllithium, trimethylsilylmethylmagnesium bromide, and combinations thereof. The alkylating agent may be utilized with a known component, such as a solvent, e.g., diethyl ether.

Reacting the intermediate dichloride compound with the alkylating agent makes the alkylated metallocene having one or more tetrahydropentalenyl groups.

One or more embodiments, e.g., when the chloride compound includes zirconium, provide that the alkylated metallocene having one or more tetrahydropentalenyl groups can be represented by the following formula: 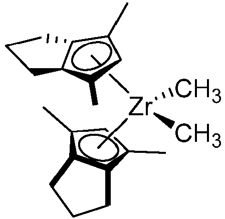 This alkylated metallocene includes two tetrahydropentalenyl groups.

One or more embodiments, e.g., when the chloride compound includes hafnium, provide that the alkylated metallocene having one or more tetrahydropentalenyl groups can be represented by the following formula: 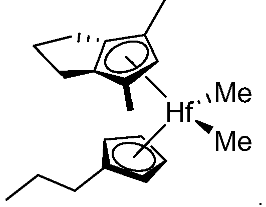 This alkylated metallocene includes one tetrahydropentalenyl group.

As mentioned, the alkylated metallocenes having one or more tetrahydropentalenyl groups can be made without isolating the intermediate compound from the reaction mixture. In other words, the alkylated metallocenes having one or more tetrahydropentalenyl groups can be made without isolating the intermediate compound from the reaction mixture prior to reacting the intermediate compound and the alkylating agent. Reacting the chloride compound with the lithium compound to make a reaction mixture comprising the intermediate dichloride compound; and reacting the intermediate dichloride compound with an alkylating agent to make the alkylated metallocene having one or more tetrahydropentalenyl group occur in a same reaction vessel. Utilizing a same reaction vessel may advantageously circumvent a number of purification procedures, help to minimize chemical waste, and/or help to reduce synthesis time of a target molecule, e.g. the alkylated metallocenes having one or more tetrahydropentalenyl groups discussed herein. While not wishing to be bound to theory, it is believed that prior methods utilized multiple reaction vessels, i.e. and isolated intermediates, due to the generation of byproducts, the generation of side-products, a desire to employ different solvents for different reaction steps, and/or unfeasibly low product yields.

The alkylated metallocene having one or more tetrahydropentalenyl groups may be utilized for a number of applications. For instance, the alkylated metallocene having one or more tetrahydropentalenyl groups may be utilized to make polymerization catalysts, which may be referred to as metallocene catalysts. Metallocene catalysts are known in the art. For instance, the alkylated metallocene having one or more tetrahydropentalenyl groups may be contacted, under activating conditions, with an activator so as to activate the alkylated metallocene having one or **more tetrahydropentalenyl** groups, thereby making the polymerization catalyst. Activating conditions are well known in the art.

The polymerization catalyst, which is made from the alkylated metallocene having one or more tetrahydropentalenyl groups can be utilized to make a polymer. For instance, the polymerization catalyst and an olefin can be contacted under polymerization conditions to form a polymer, e.g., a polyolefin polymer. The polymerization process may be a suspension polymerization process, a slurry polymerization process, and/or a gas phase polymerization process. The polymerization process may utilize using known equipment and reaction conditions, e.g., known polymerization conditions. The polymerization process is not limited to any specific type of polymerization system. The polymer can be utilized for a number of articles such as films, fibers, nonwoven and/or woven fabrics, extruded articles, and/or molded articles.

Embodiments provide that the chloride compound and the lithium compound may be reacted at from 1.0 to 2.7 molar equivalents of the lithium compound to 1 molar equivalent of the chloride compound. One or more embodiments provide that the chloride compound and the lithium compound are reacted at from 1.1 to 2.0 molar equivalents of the lithium compound to 1 molar equivalent of the chloride compound. Embodiments provide that the chloride compound and the lithium compound may be reacted at temperature from -50 °C to 75 °C. NMR (nuclear magnetic resonance spectroscopy) analysis can be used to monitor formation of the intermediate dichloride compound. Other known reaction components and/or conditions may be utilized.

Embodiments provide that the intermediate dichloride compound and the alkylating agent may be reacted at from 2.0 to 3.0 molar equivalents of the alkylating agent to 1 molar equivalent of the intermediate dichloride compound. One or more embodiments provide that the intermediate dichloride compound and the alkylating agent may be reacted at from 2.2 to 2.6 molar equivalents of the alkylating agent to 1 molar equivalent of the intermediate dichloride compound. Embodiments provide that the intermediate dichloride compound and the alkylating agent may be reacted at temperature from -50 °C to 75 °C. Other known reaction components and/or conditions may be utilized.

Embodiments provide that the lithium compound can be made by reacting butyl lithium with a bicyclic alkene compound. Butyl lithium is known in the art and may be obtained commercially. The bicyclic alkene compound can be represented by the following formula: 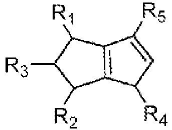 where wherein R₁, R₂, R₃, R₄, and R₅ are as previously discussed, i.e., each is independently H or a (C1-C4) alkyl.

Embodiments provide that the butyl lithium and the bicyclic alkene compound may be reacted at from 1.0 to 2.5 molar equivalents of the butyl lithium to 1 molar equivalent of the bicyclic alkene compound. One or more embodiments provide that the butyl lithium and the bicyclic alkene compound are reacted at from 1.1 to 1.6 molar equivalents of the butyl lithium to 1 molar equivalent of the bicyclic alkene compound. Embodiments provide that the butyl lithium and the bicyclic alkene compound may be reacted at temperature from -0 °C to 50 °C. Other known reaction components and/or conditions may be utilized.

Embodiments provide that the bicyclic alkene compound can be made by reacting a bicyclic ketone compound with an alkylating agent and diethyl ether to form a bicyclic alcohol compound and then dehydrating the bicyclic alcohol compound to make the bicyclic alkene compound. The bicyclic ketone compound can be represented by the following formula: 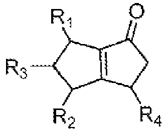 where R₁, R₂, R₃, and R₄, are as previously discussed, i.e., each is independently H or a (C1-C4) alkyl. The alkylating agent can be as previously discussed.

Embodiments provide that the bicyclic ketone compound and the alkylating agent may be reacted at from 1.0 to 2.0 molar equivalents of the alkylating agent to 1 molar equivalent of the bicyclic ketone compound. One or more embodiments provide that the bicyclic ketone compound and the alkylating agent may be reacted at from 1.1 to 1.6 molar equivalents of the alkylating agent to 1 molar equivalent of the bicyclic ketone compound. Embodiments provide that the bicyclic ketone compound and the alkylating agent may be reacted at temperature from 10 °C to 50 °C. Other known reaction components and/or conditions may be utilized.

The bicyclic alcohol compound can be represented by the following formula: 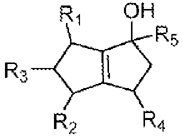 where R₁, R₂, R₃, R₄, and R₅ are as previously discussed, i.e., each is independently H or a (C1-C4) alkyl.

As mentioned, the bicyclic alcohol compound can be dehydrated to make the bicyclic alkene compound. The bicyclic alcohol compound can be dehydrated by a known process, e.g., by utilizing known components and known conditions. For instance, the bicyclic alcohol compound can be dehydrated by reacting with HCl, among other known dehydrating reagents. For example, 0.6 Molar (M) or higher hydrochloric acid (aqueous HCl) or anhydrous HCl in an organic solvent such as ethanol, tetrahydrofuran or toluene may be utilized. The hydrochloric acid may be from 0.2 M to 12 M, alternatively from 0.6 M to 6 M. One or more embodiments provide that the bicyclic alcohol compound can be dehydrated utilizing p-Toluenesulfonic acid. For example, 0.1 mol% to 20 mol% (based on 1 equivalent of bicyclic alcohol compound) of p-Toluenesulfonic acid may be utilized. One or more embodiments provide that 2 mol% to 10 mol% (based on 1 equivalent of bicyclic alcohol compound) of p-Toluenesulfonic acid is utilized; one or more embodiments provide that 5 mol% (based on 1 equivalent of bicyclic alcohol compound) of p-Toluenesulfonic acid is utilized.

Embodiments provide that the bicyclic ketone compound can be made by reacting a cyclic alkene compound with an acid compound in the presence of an aprotic solvent and an effective amount of a phosphoric and/or sulfonic acid reagent.

The cyclic alkene compound can be represented by the following formula: 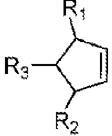 where R₁, R₂, and R₃, are as previously discussed, i.e., each is independently H or a (C1-C4) alkyl. The cyclic alkene compound can be obtained commercially. The cyclic alkene compound may be synthesized by a known process, e.g., by utilizing known components and known conditions.

The acid compound can be represented by the following formula: 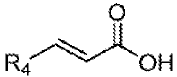 where R₄ is as previously discussed, i.e., R₄ is H or a (C1-C4) alkyl. The acid compound can be obtained commercially. The acid compound may be synthesized by a known process, e.g., by utilizing known components and known conditions. One or more embodiments provide that the acid compound is (E)-2-butenoic acid, which may be referred to as crotonic acid.

Embodiments provide that the cyclic alkene compound and the acid compound may be reacted at a molar ratio from 1:1 to 1.5:1 moles of cyclic alkene compound to moles acid compound. Embodiments provide that the cyclic alkene compound and the acid compound may be reacted at temperature from -10 °C to 75 °C. Other known reaction components and/or conditions may be utilized.

As mentioned, the bicyclic ketone compound can be made by reacting a cyclic alkene compound with an acid compound in the presence of an aprotic solvent. The aprotic solvent can be a polar aprotic solvent and/or a dipolar aprotic solvent. The aprotic solvent may be selected from sulfolane, dipropylsulfone, 1,2-dimethoxyethane, diethylene glycol dimethyl ether, tetraethylene glycol dimethyl ether, dichloromethane, diethyl ether, and mixtures of any two or more thereof; sulfolane is a dipolar aprotic solvent and dipropylsulfone, 1,2-dimethoxyethane, diethylene glycol dimethyl ether, tetraethylene glycol dimethyl ether, dichloromethane, diethyl ether are polar aprotic solvents. One or more embodiments provide that the aprotic solvent can be a sulfone, an ether (including glycol ethers), an organo chloride, or a combination thereof.

Embodiments provided that the bicyclic ketone compound and the cyclic alkene compound are reacted in the absence of a cyclic ether. In other words, the aprotic solvent excludes, i.e., does not include, cyclic ethers. Examples of cyclic ethers include tetrahydrofuran and dioxane.

The aprotic (polar and/or dipolar) solvent may have a relative polarity that is above 0.099 and is below 0.444. All individual values and subranges from above 0.099 to below 0.444 are included; for example, the aprotic solvent can have a relative polarity from 0.117 to 0.410, 0.231 to 0.309. The relative polarity of solvents is readily available from published materials, such as Christian Reichardt, Solvents and Solvent Effects in Organic Chemistry, Wiley-VCH Publishers, 3rd ed., 2003, to which the relative polarity herein refers.

Utilizing the aprotic solvent, as discussed herein, when reacting the cyclic alkene compound with the acid compound to make the bicyclic ketone compound can advantageously provide a homogenous reaction mixture. The homogenous reaction mixture can indicate solvent stability, which is desirable for this reaction.

Utilizing the aprotic solvent, as discussed herein, when reacting the cyclic alkene compound with the acid compound to make the bicyclic ketone compound can advantageously provide an area count of product determined by GC/MS greater than 1,000,000. For instance, the area count of product determined by GC/MS can be from 1,000,000 to 10,000,000, or from 1,500,000 to 6,000,000. An area count of product determined by GC/MS greater than 1,000,000 can indicate desirable product formation. GC/MS Analysis can be determined as follows. A concentration of desired product can be determined using a gas chromatography system comprising an Agilent Technologies 7890A GC system equipped with an analytical column (Agilent 19091S-433 HP-5ms 0 °C - 325 °C: 15 m x 250 µm x 0.25 µm) using Helium/Hydrogen as carrier gas, coupled with a mass detector comprising an Agilent Technologies 5977B MSD analyzer operating in electronic impact (EI) mode. The raw data can be analyzed by performing ion extraction of the desired product molecular weight, e.g., 136 g/mol, and the resulting spectra can integrated to obtain the area counts.

As mentioned, the bicyclic ketone compound can be made by reacting a cyclic alkene compound with an acid compound in the presence of an effective amount of a phosphoric and/or sulfonic acid reagent.

In some embodiments the sulfonic acid reagent is, or consists essentially of, a P₂O₅/H₃CSO₃H mixture, or a reaction product thereof. Alternatively the sulfonic acid reagent may consist essentially of an alkylsulfonic acid such as a (C1-C6)alkylsulfonic acid such as methanesulfonic acid. The expression "consist essentially of the P₂O₅/H₃CSO₃H mixture" means the sulfonic acid reagent are free of polyphosphoric acid (PPA). In some aspects the P₂O₅/H₃CSO₃H mixture is a 0.1/1 (weight/weight) P₂O₅/H₃CSO₃H mixture, known as Eaton's reagent.

Methanesulfonic acid is a compound of formula H₃CSO₃H and has CAS number 75-75-2 and is widely available from commercial suppliers.

Mixture of a phosphorous pentoxide and methanesulfonic acid or P2O5/H3CSO3H mixture is a blend or reaction product of phosphorous pentoxide and methane sulfonic acid. The weight/weight ratio of P₂O₅/H₃CSO₃H in the mixture may be from 0.05 to 1 alternatively 0.075 to 1, alternatively 0.077 to 1, alternatively 0.1 to 1, and alternatively 0.2 to 1. The 0.075/1 (wt/wt) P₂O₅/H₃CSO₃H mixture is commercially available and, again, may be referred to as Eaton's reagent. The mixture of P₂O₅ and CH₃SO₃H may be formed in situ, e.g., in the presence of one or more reactants, such as prior to or during a reaction discussed herein. Alternatively, the mixture of P₂O₅ and CH₃SO₃H may be preformed prior to performing the reaction. It may be convenient to prepare the P₂O₅/H₃CSO₃H mixture before performing other reactions, and store the resulting mixture for later use in embodiments of the present disclosure.

A sulfonic acid reagent is an acidic material having O-P(O)-OH acid groups and/or C-S(O)₂-OH acid groups, or an acidic reaction product thereof. The sulfonic acid reagent may be, or may consist essentially of, a mixture of a phosphorous pentoxide and methanesulfonic acid ("P₂O₅/H₃CSO₃H mixture"), or a reaction product thereof. In some embodiments the sulfonic acid reagent consists essentially of the P₂O₅/H₃CSO₃H mixture. Alternatively the sulfonic acid reagent may consist essentially of an alkylsulfonic acid such as a (C1-C6)alkylsulfonic acid such as methanesulfonic acid. The expression "consist essentially of" means the phosphoric and/or sulfonic acid reagent, and the reaction utilized therein, are free of PPA.

Polyphosphoric acid or PPA has CAS no. 8017-16-1 and is a compound generally of formula HO-[P(=O)(OH)]n-H, wherein subscript n indicates degree of polymerization.

Phosphorous pentoxide is a compound of formula P₂O₅ and has CAS number 1314-56-3 and is widely available from commercial suppliers.

In some aspects each reactant, reagent, solvent, or other material used in the embodiments herein, and each product thereof, is free of Pt, Ni, Pd, Rh, and Ru.

An effective amount is a quantity sufficient for enabling the making of a detectable amount of intended product. An effective amount of the phosphoric and/or sulfonic acid reagent is a quantity thereof sufficient for enabling the making of a **detectable** amount of the bicyclic ketone compound. Detectable amounts may be detected, and optionally characterized, by any suitable analytical method such as 1 H-nuclear magnetic resonance (1 H-NMR), high performance liquid chromatography (HPLC, versus a known standard), gas chromatography (GC, versus a known standard), or mass spectrometry; typically 1 H-NMR. The effective amount of the phosphoric and/or sulfonic acid reagent used in making the bicyclic ketone compound may vary depending upon its composition, reaction conditions, and costs. A skilled person may readily determine an effective amount thereof by starting with an initial reaction mixture of reactants, and 90 wt% of the phosphoric and/or sulfonic acid reagent, and thereafter systematically try reaction mixtures containing lower wt% of the phosphoric and/or sulfonic acid reagent until an optimal result under the reaction conditions is found. When the phosphoric and/or sulfonic acid reagent is polyphosphoric acid (PPA), the P₂O₅/H₃CSO₃H mixture, or the combination of PPA and P₂O₅/H₃CSO₃H mixture, the effective amount may be from 55 to 90 wt%, alternatively from 60 to 85 wt% based on total weight of reactants, and the phosphoric and/or sulfonic acid reagent. Alternatively, the effective amount of the P₂O₅/H₃CSO₃H mixture may be from 1 to 10 mole equivalents, alternatively 1 to 5 mole equivalents, alternatively 1 to 3 mole equivalents thereof relative to the number of moles of cyclic alkene compound. For example, if 1 .0 mole of cyclic alkene compound is used, then the effective amount of the P₂O₅/H₃CSO₃H mixture may be from 1 to 10 molar equivalents, alternatively from 1 to 5 molar equivalents, alternatively from 1 to 3 molar equivalents.

As used herein, an "alkyl" includes linear, branched and cyclic paraffin radicals that are deficient by one hydrogen. Thus, for example, CH₃ ("methyl") and CH₂CH₃ ("ethyl") are examples of alkyls.

As used herein, an "alkylene" includes linear, branched and cyclic hydrocarbon radicals deficient by two hydrogens. Thus, CH₂ ("methylene") and CH₂CH₂ ("ethylene") are examples of alkylene groups. Other groups deficient by two hydrogen radicals include "arylene" and "alkenylene".

A number of aspects of the present disclosure are provided as follows.

Aspect 1 provides a method of making an alkylated metallocene having one or more tetrahydropentalenyl groups, the method comprising: reacting a chloride compound with a lithium compound to make a reaction mixture comprising an intermediate dichloride compound; and reacting the intermediate dichloride compound with an alkylating agent to make the alkylated metallocene having one or more tetrahydropentalenyl groups, wherein the dichloride compound is not isolated from the reaction mixture, wherein the lithium compound is represented by: 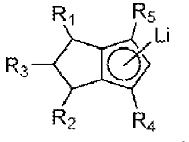 wherein R1, R2, R3, R4, and R5 are each independently H or a (C1-C4) alkyl.

Aspect 2 provides the method of aspect 1, wherein reacting the chloride compound with the lithium compound to make the reaction mixture comprising the intermediate dichloride compound occurs in absence of the alkylating agent.

Aspect 3 provides the method of any one of aspects 1-2 wherein the chloride compound includes 3 or 4 chlorine atoms.

Aspect 4 provides the method of any one of aspects 1-3, wherein the chloride compound is selected from zirconium (IV) chloride and n-(propylcyclopentadienyl)hafniumtrichloride (dimethoxyethane).

Aspect 5 provides the method of any one of aspects 1-4, wherein the intermediate dichloride compound includes one or more tetrahydropentalenyl groups.

Aspect 6 provides the method of any one of aspects 1-5, wherein the alkylating agent is an alkylmagnesium halide.

Aspect 7 provides the method of any one of aspects 1-6, wherein the lithium compound is made by reacting butyl lithium with a bicyclic alkene compound represented by:

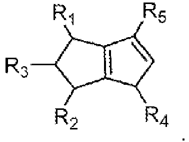

Aspect 8 provides the method of aspect 7, wherein the bicyclic alkene compound is made by reacting a bicyclic ketone compound with an alkylating agent and diethyl ether to form a bicyclic alcohol compound and dehydrating the bicyclic alcohol compound to make the bicyclic alkene compound, wherein the bicyclic ketone compound is represented by:

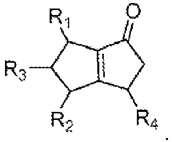

Aspect 9 provides the method of aspect 8, wherein the bicyclic ketone compound is made by reacting a cyclic alkene compound represented by: 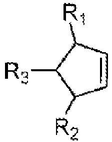 with an acid compound represented by: 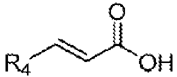 in the presence of an aprotic solvent and an effective amount of a phosphoric and/or sulfonic acid reagent and under reaction conditions sufficient to make the bicyclic ketone compound.

Aspect 10 provides the method of aspect 9, wherein the aprotic solvent has a relative polarity above 0.099 and below 0.444.

Aspect 11 provides the method of any one of aspects 9-10, wherein the phosphoric and/or sulfonic acid reagent is a P₂O₅/H₃CSO₃H mixture.

### EXAMPLES

Unless noted otherwise herein, use the following preparations for characterizations. Carry out syntheses under an atmosphere of dry nitrogen in a glovebox when indicated. Perform reactions requiring anhydrous conditions under an atmosphere of dry nitrogen in oven-dried glassware cooled under a stream of dry nitrogen. Anhydrous toluene, hexanes, tetrahydrofuran, diethyl ether and 1 ,2-dimethoxyethane are from Sigma-Aldrich. Sulfolane, dipropylsulfone, DMSO, 1,2-dimethoxyethane, diethylene glycol dimethyl ether, tetraethylene glycol dimethyl ether, dichloromethane, ethanol, tert-butanol, tetrahydrofuran, 1,4-dioxane and diethyl ether were purchased from Sigma-Aldrich or Fisher. Solvents that are used for experiments performed in a nitrogen-filled glovebox are further dried by storage over activated 4 Angstrom molecular sieves. Zirconium (IV) chloride (ZrCl₄) and Hafnium (IV) were purchased from Boulder Scientific and STREM and are used as received. All other reagents are purchased from Sigma-Aldrich and are used as received. For example, P₂O₅/CH₃SO₃H (7.7 wt% P₂O₅) may be purchased from Sigma- Aldrich, CAS# 39394-84-8 and P₂O₅/CH₃SO₃H ( 7.5 wt% P₂O₅) from vendor Frontier Scientific may be purchased from Fisher Scientific, CAS# 39394-84-8.

¹H-NMR (proton nuclear magnetic resonance spectroscopy) chemical shift data are reported in parts per million (ppm) down field relative to tetramethylsilane (TMS), δ scale, using residual protons in deuterated solvent as references. The ¹H-NMR chemical shift data measured in CDCl₃ are referenced to 7.26 ppm, data measured in benzene-d6 (C₆D₆) to 7.16 ppm, data measured in tetrahydrofuran-d8 (THF-d8) to 3.58 ppm. ¹H-NMR chemical shift data are reported in the format: chemical shift in ppm (multiplicity, coupling constant(s) in Hertz (Hz), and integration value. Multiplicities are abbreviated s (singlet), d (doublet), t (triplet), q (quartet), pent (pentet), m (multiplet), and br (broad).

GC/MS (EI) refers to gas chromatography-mass spectrometry (electron ionization).

### GC-MS Analysis:

The concentration of desired product in the crude organic product is performed using a gas chromatography system comprising an Agilent Technologies 7890A GC system equipped with an analytical column (Agilent 19091S-433 HP-5ms 0 °C - 325 °C: 15 m x 250 µm x 0.25 µm) using Helium/Hydrogen as carrier gas, coupled with a mass detector comprising an Agilent Technologies 5977B MSD analyzer operating in electronic impact (EI) mode. The raw data is analyzed by performing ion extraction of the desired product molecular weight (136 g/mol), the resulting spectra are integrated and the area counts are reported below. In the following examples 1 Oz = 28,3495 g and 1 torr = 133.322 Pa.

Example 1 synthesis of a metallocene having a tetrahydropentalenyl group, was performed as follows
Synthesis of a bicyclic ketone compound, was performed as follows. The bicyclic ketone compound can be represented by the following formula: 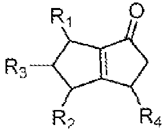 wherein R₁, R₂, R₃, and R₄, are each independently H or a (C1-C4) alkyl.

A cyclic alkene compound that can be represented by the following formula (cyclopentene; R₁, R₂, and R₃ were each H): 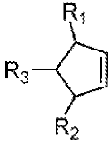 and an acid compound that can be represented by the following formula ((E)-2-butenoic acid; R₄ was CH3): 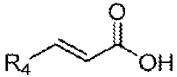 were utilized. In a fume hood under a nitrogen atmosphere, an acid compound ((E)-2-butenoic acid; 40.0 g; 464.63 mmol) and a cyclic alkene compound (cyclopentene; 41.05 mL, 464.63 mmol) were added to a container (1 liter round bottom flask equipped with a stir bar). Next, an aprotic polar solvent (sulfolane; 150 m; relative polarity 0.41) was added to the contents of the container. The contents of the container were cooled to 0 °C. Next, P₂O₅/H₃CSO₃H mixture (7.5 wt% P₂O₅) was added dropwise (221.2 mL, 1393.9 mmol) at 0 °C to the container. While stirring, the contents of the container were warmed up to approximately 20 °C and then stirring was continued for 72 hours. The resulting crude product was diluted with water (40 mL). Next, the contents of the container were diluted with diethyl ether (200 mL). The reaction was slowly quenched with sodium hydroxide (680 mL) in the following concentrations and order of addition: 1 N NaOH (50 mL), 2 N NaOH (50 mL), 2.5 N NaOH (270 mL) and 3 N NaOH (310 mL). The contents of the container were tested until it reached pH 8 to pH 9 upon addition of NaOH. The aqueous and organic layers were separated in a separatory funnel. The aqueous layer was extracted three times with diethyl ether (3 × 160 mL). The organic layers were combined and washed with 0.5 N NaOH (300 mL). Followed by a wash with brine (150 mL), dried over anhydrous magnesium sulfate and filtered. The solvent was removed with vacuum to provide the bicyclic ketone compound (53.76; observed as a dark brown liquid product; 85% yield). The bicyclic ketone compound (where R₁, R₂, and R₃ were each H, and R₄ was CH₃) was characterized by 1H-NMR and GC/MS. 1H-NMR (400 MHz, Chloroform-d) δ 3.05 - 2.88 (m, 1H), 2.88 - 2.71 (m, 1H), 2.66 - 2.46 (m, 1H), 2.46 - 2.28 (m, 3H), 2.27 - 2.15 (m, 1H), 2.15 - 1.98 (m, 1H), 1.97 - 1.43 (m, 2H), 1.22 - 1.13 (m, 3H).

Synthesis Number One of a bicyclic alkene compound, that can be represent by the following formula, was performed as follows

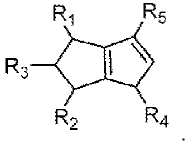

Methyl magnesium bromide solution (3 M in diethyl ether; 76.36 mL; 229.1 mmol) was added to a first container (1 liter, 2-neck round bottom reaction flask) under an atmosphere of dry nitrogen, followed by diethyl ether (80 mL). The contents of the first container were cooled to 0 °C solution and then stirred for 15 minutes. The bicyclic ketone compound (24 g, 176.22 mmol) was dissolved in anhydrous diethyl ether (190 mL) in a second container (250 mL round bottom flask). Then the contents of the second container were added to the contents of the first container over 30 minutes while maintain the temperature below 10 °C. Then, the contents of the first container were stirred for 20 hours at approximately 20 °C to provide a mixture containing a bicyclic alcohol compound that can be represented by the following: 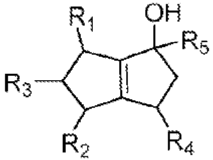 where R₁, R₂, and R₃ were each H, and R₄ and R₅ were each CH₃.

The bicyclic alcohol compound of was not isolated or characterized by 1H-NMR. Then, the bicyclic alcohol compound was dehydrated by the addition of aqueous HCl (0.6 M; 300 mL) at a temperature of approximately - 10 °C to the contents of the first container, followed by addition of aqueous HCl (6M; 50 mL) also at a temperature of approximately - 10 °C to the contents of the first container; the total HCL addition was over approximately 1.5 hours. Then, the contents of the container were stirred vigorously for 5 hours at approximately 20 °C. The organic phase was separated, and the aqueous layer was extracted with diethyl ether (2 x 150 mL). The combined organic layers were washed with saturated NaHCO₃ (150 mL) and brine (150 mL). The organic layers were dried over magnesium sulfate and filtered, and the solvent was removed with vacuum. The product was passed through a silica gel plug and eluted with diethyl ether/hexane to provide the bicyclic alkene compound (20.2 g; 85%; where R₁, R₂, and R₃ were each H, and R₄ and R₅ were each CH₃). The bicyclic alkene compound was obtained as a mixture of double bond regioisomers. 1H-NMR (400 MHz, Chloroform-d) δ 3.07 (p, J = 2.7 Hz, 2H), 2.89 - 2.69 (m, 3H), 2.62 (q, J = 8.1, 7.2 Hz, 1H), 2.33 - 2.17 (m, 7H), 2.17 - 2.02 (m, 3H), 1.98 - 1.83 (m, 8H), 1.83 - 1.63 (m, 12H), 1.58 (s, 5H).

Synthesis Number Two of a bicyclic alkene compound, that can be represent by the following formula, was performed as follows

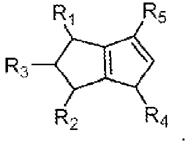

A 3-necked flask (100 mL) was fitted with a rubber septa and a Nitrogen-inlet; a stir bar was added under N₂. The flask was evacuated under vacuum and refilled with N₂ three times. The flask was charged with MeMgBr in diethyl ether (3.0 M; 9.5 mmol; 3.2 mL). Then, the flask was immersed in an ice bath before adding distilled enone (1.0 g; 7.3 mmol) in diethyl ether (19 mL) via a syringe dropwise (rate of addition was 20 mL/hr). Then, an aliquot of the reaction mixture was quenched with a few drops of methanol, diluted with ethyl acetate and analyzed by gas-chromatography and monitored for presence of enone. After the enone was consumed, the reaction was maintained at 0 °C and quenched with NH₄Cl (10wt/vol%; 1.3 equiv; 5.1mL) that was added over 20 min (rate of addition was 20 mL/h). The bath was continued to be maintained at 0 °C during the addition; the reaction was stirred for 1 hr to provide a bicyclic alcohol compound that can be represented by the following: 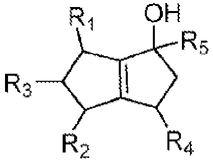 where R₁, R₂, and R₃ were each H, and R₄ and R₅ were each CH₃.

Then, p-Toluenesulfonic acid (70 mg, 0.4 mmol, 5 mol% based upon 1 equivalent of the bicyclic alcohol compound) was added and the reaction was monitored by TLC. When the allylic alcohol was completely consumed, the organic layer was washed with saturated aqueous NaHCO₃ (2x10 mL); then, the organic layer was dried over MgSO₄ and a rotary evaporator to provide the bicyclic alkene compound (0.79 g, 80% yield) where R₁, R₂, and R₃ were each H, and R₄ and R₅ were each CH₃. Synthesis of a lithium compound that can be represented by the following, was performed as follows

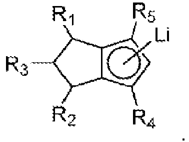

In a glove box, in a container, the bicyclic alkene compound, as made in Synthesis Number One, (20.2 g; 150.5 mmol) was dissolved in hexanes (350 mL) while stirring. A solution of n-butyl lithium in hexanes (2.5 M; 78.26 mL;195.65 mmol) was added dropwise to the container and the contents of the container were stirred for 20 hours at approximately 20 °C. Then, the contents of the container were collected by vacuum filtration, and then washed with hexanes and dried under vacuum to provide the lithium compound (6.89 g; 33% yield; where R₁, R₂, and R₃ were each H, and R₄ and R₅ were each CH₃). 1H-NMR (400 MHz, THF-*d*₈) δ 5.13 (s, 1H), 2.43 (t, *J =* 6.8 Hz, 4H), 2.20 - 2.08 (m, 2H), 1.95 (s, 6H).This metallocene having the tetrahydropentalenyl group can be represented by the following:

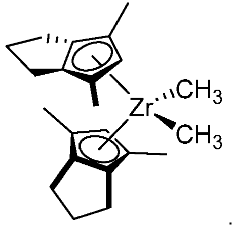

In a drybox, a chloride compound (zirconium (IV) chloride; 3.32 g; 14.27 mmol) and toluene (330 mL) were added to a container (32-oz glass jar) and stirred and cooled to -33 °C for 45 minutes. The lithium compound (5.0 g; 35.67 mmol) was added to the contents of the container in several portions. Then, the contents of the container were stirred for 24 hours at approximately 20 °C to make a reaction mixture. NMR analysis was used to monitor formation of an intermediate compound, i.e. bis(4,6-dimethyl-1,2,3,4-tetrahydropentalenyl)zirconium dichloride, in the reaction mixture by removing an aliquot from the container. 1H NMR (400 MHz, Benzene-*d*₆) δ 5.39 (d, *J* = 8.4 Hz, 2H), 3.09 (ddd, *J* = 14.6, 8.6, 1.6 Hz, 4H), 2.61 - 2.43 (m, 2H), 2.38 (dt, J = 14.6, 8.6 Hz, 4H), 2.09 - 2.00 (m, 2H), 1.75 (s, 12H). Then, without isolating the intermediate compound, a solution of methyl magnesium bromide in diethyl ether (3.0 M; 9.51 mL; 228.54 mmol) was added dropwise to the contents of the container, which were stirred for 24 hours at approximately 20 °C. The contents of the container were filtered, and solvent was removed under vacuum. Next, the resultant solid product was dissolved in hexanes (500 mL) and stirred for 3 hours. Thereafter, the resultant solid product was again filtered, and solvent was removed under vacuum to provide the metallocene having a tetrahydropentalenyl group, as indicated above,(3.54 g; 64% yield; which may be referred to as bis(4,6-dimethyl-1,2,3,4-tetrahydropentalenyl)zirconium dimethyl). 1H NMR (400 MHz, Benzene-*d*₆) δ 4.83 (s, 2H), 2.71 (ddd, *J* = 14.5, 8.0, 3.3 Hz, 4H), 2.57 (dt, J = 14.7, 8.7 Hz, 4H), 2.22 - 2.08 (m, 4H), 1.72 (s, 12H), -0.39 (s, 6H). The yield is reported in Table 1.

Comparative Example A was performed as follows. In drybox, zirconium (IV) chloride (0.125 g; 0.54 mmol) and toluene (8 mL) were added to a first container (2-oz glass jar,) and stirred and cooled to -33 °C for 30 minutes. A solution of methyl magnesium bromide in diethyl ether (3.0 M; 0.358 mL;1.073 mmol) was cooled to -33 °C for 30 minutes in a second container (20 mL vial). Next, the contents of the first container were added to the contents of the second container, which was maintained -33 °C for 30 min. Next, the lithium compound (0.150 g, 1.073 mmol) was added to the contents of the second container in several portions while stirring; the stirring was continued for 24 hours at approximately 20 °C. Then, solvent was removed under vacuum. Next, the resulting solid product was dissolved in hexanes (30 mL) and filtered. Solvent was removed under vacuum to provide bis(4,6-dimethyl-1,2,3,4-tetrahydropentalenyl)zirconium dimethyl (0.052 g; 25% yield). 1H NMR 4.83 (s, 2H), 2.72 (ddd, J = 14.4, 7.9, 3.4 Hz, 4H), 2.57 (dt, J = 14.7, 8.7 Hz, 4H), 2.23 - 2.08 (m, 4H), 1.72 (s, 12H), -0.39 (s, 6H). In addition to the bis(4,6-dimethyl-1,2,3,4-tetrahydropentalenyl)zirconium dimethyl, the presence of additional byproducts was observed by NMR analysis. The yield is reported in Table 1.

Comparative Example B was performed as follows. In drybox, zirconium (IV) chloride (0.125 g, 0.54 mmol) and toluene (10 mL) were added to a first container (2-oz glass jar,) and stirred and cooled to -33 °C for 30 minutes. A solution of methyl magnesium bromide in diethyl ether (3.0 M; 0.358 mL;1.073 mmol) was cooled to -33 °C for 30 minutes in a second container (20 mL vial). Next, the contents of the first container were added to the contents of the second container, and the combined contents were very quickly brought to approximately 20 °C and stirred for 15 minutes at that temperature. Next, the lithium compound (0.150 g; 1.073 mmol) was added to the contents of the second container in several portions while stirring; the stirring was continued for 24 hours at approximately 20 °C. Then, solvent was removed under vacuum. Next, the resulting solid product was dissolved in hexanes (30 mL) and filtered. Solvent was removed under vacuum to provide bis(4,6-dimethyl-1,2,3,4-tetrahydropentalenyl)zirconium dimethyl (0.055 g; 26% yield) 1H NMR (400 MHz, Benzene-d6) δ 4.83 (s, 2H), 2.72 (ddd, *J* = 14.4, 7.9, 3.4 Hz, 4H), 2.57 (dt, *J* = 14.7, 8.7 Hz, 4H), 2.23 - 2.08 (m, 4H), 1.72 (s, 12H), -0.39 (s, 6H).. The yield is reported in Table 1. In addition to the bis(4,6-dimethyl-1,2,3,4-tetrahydropentalenyl)zirconium dimethyl, the presence of additional byproducts, e.g., byproducts A and B shown below, were observed by NMR analysis. 1H NMR (400 MHz, Benzene-*d*₆) δ 5.60 (s, 1H), 5.50 (s, 0.26H), 2.41 - 2.32 (m, 4H), 2.03 (s, 1H), 2.01 - 1.94 (m, 2H), 1.80 (br s, 6H), 0.49 (s, 2H), 0.37 (s, 9H).

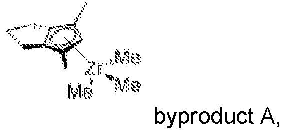

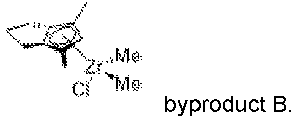

Comparative Example C was performed as follows. In drybox, zirconium (IV) chloride (0.113 g; 0.485 mmol) and toluene (7 mL) were added to a container (2-oz glass jar,) and stirred. Next, the lithium compound (0.150 g; 1.067 mmol) was added to the contents of the container in several portions while stirring. Next, a solution of methyl magnesium bromide in diethyl ether (3.0 M; 0.356 mL;1.067 mmol) was added dropwise to the contents of the container, which were stirred for 24 hours at approximately 20 °C. Then, solvent was removed under vacuum. Next, the resulting solid product was dissolved in hexanes (15 mL) and filtered. Solvent was removed under vacuum to provide bis(4,6-dimethyl-1,2,3,4-tetrahydropentalenyl)zirconium dimethyl (0.058 g; 30% yield). 1H NMR (400 MHz, Benzene-d6) δ 4.83 (s, 2H), 2.72 (ddd, J = 14.4, 7.9, 3.4 Hz, 4H), 2.57 (dt, J = 14.7, 8.7 Hz, 4H), 2.23 - 2.08 (m, 4H), 1.72 (s, 12H), -0.39 (s, 6H). In addition to the bis(4,6-dimethyl-1,2,3,4-tetrahydropentalenyl)zirconium dimethyl, the presence of additional byproducts was observed by NMR analysis. The yield is reported in Table 1.

**Table 1**

| | Yield |
|---|---|
| | (%) |
| Example 1 | 64 |
| Comparative Example A | 25 |
| Comparative Example B | 26 |
| Comparative Example C | 30 |

The data of Table 1 illustrate that Example 1 has an improved, i.e., greater, yield as compared to Comparative Examples A-C.

The data of Table 1 illustrate that Example 1 has an improved, i.e., reduced, generation of byproducts, as shown by the improved yield, as compared to Comparative Examples A-C.

Bis(n-propylcyclopentadienyl)hafnium dichloride (25.0 g, 53.91 mmol) was melted at 140 °C and HfCl₄ (17.27 g, 53.91 mmol) was added. After stirring for about 30 min, a bulb-to-bulb distillation apparatus was attached and the pot was heated to approximately 135 °C. (PrCp)HfCl₃ was distilled in about 1 hour at approximately 110 °C and 0.4 torr, as an off-white colored solid with traces of a tan colored contaminant. After cooling to room temperature, the distillate was dissolved in toluene (50 mL) and DME (45 mL) at 80 °C to form a clear solution. This product was cooled to room temperature and then placed in the freezer. The product was collected by filtration, washing with hexane (3 x 20 mL) and then dried under vacuum. N-propylcyclopentadienyl) hafnium trichloride (dimethoxyethane) was obtained as a crystalline white solid (39.79 g ) in 76.5 % yield. 1H NMR (400 MHz, Benzene-*d*₆) δ 6.22 (t, *J* = 2.8 Hz, 2H), 6.15 (t, *J* = 2.7 Hz, 2H), 3.57 - 3.06 (m, 4H), 3.06 - 2.98 (m, 2H), 1.58 (dq, *J* = 14.9, 7.4 Hz, 2H), 0.86 (t, *J* = 7.4 Hz, 3H).

N-propylcyclopentadienyl)hafniumtrichloride (dimethoxyethane) can be represented by the following formula:

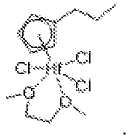

Example 2 synthesis of a metallocene having a tetrahydropentalenyl group, was performed as follow. This metallocene having the tetrahydropentalenyl group can be represented by the following formula:

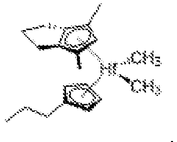

In a drybox, a chloride compound (n-propylcylopentadienyl)hafnium trichloride (dimethoxyethane) 5.50 g; 11.41 mmol) and toluene (130 mL) were added to a container (16-oz glass jar) and stirred. The lithium compound (2.0 g; 14.27 mmol) was added to the contents of the container in several portions and the contents of the container were stirred for 24 hours at approximately 20 °C to make a reaction mixture. NMR analysis was used to monitor formation of an intermediate compound, i.e. n-PrCp(4,6-dimethyl-1,2,3,4-tetrahydropentalenyl)hafnium dichloride, in the reaction mixture by removing an aliquot from the container. 1H NMR (400 MHz, Benzene-d6) δ 5.86 (t, J = 2.7 Hz, 2H), 5.71 (t, J = 2.7 Hz, 2H), 5.30 (s, 1H), 3.03 (ddd, J = 13.7, 8.4, 1.3 Hz, 2H), 2.77 - 2.67 (m, 2H), 2.44 (ddd, J = 14.3, 10.2, 8.0 Hz, 2H), 2.36 - 2.21 (m, 1H), 2.03 - 1.95 (m, 1H), 1.76 (s, 6H), 1.57 - 1.44 (m, 2H), 0.84 (td, J = 7.4, 5.5 Hz, 3H). Then, without isolating the intermediate compound, a solution of methyl magnesium bromide in diethyl ether (3.0 M; 7.61 mL; 22.83 mmol) was added dropwise to the contents of the container, which were stirred for 24 hours at approximately 20 °C. Next, solvent was removed by vacuum and the resultant solid product was dissolved in hexanes (250 mL). Hexanes were removed by vacuum to provide the metallocene having a tetrahydropentalenyl group, as indicated above, (4.79 g; 93% yield; which may be referred to as n-PrCp(4,6-dimethyl-1,2,3,4-tetrahydropentalenyl)hafnium dimethyl)). 1H NMR (400 MHz, Benzene-d6) δ 5.69 (t, J = 2.7 Hz, 2H), 5.44 (t, J = 2.6 Hz, 2H), 5.07 (s, 1H), 2.55 - 2.43 (m, 6H), 2.05 - 1.96 (m, 1H), 1.89 - 1.80 (m, 1H), 1.78 (s, 6H), 1.59 (dq, J = 14.7, 7.4 Hz, 2H), 0.97 - 0.84 (m, 3H), -0.39 (s, 6H). The yield is reported in Table 2.

Comparative Example D was performed as follows. In a drybox, n-Propylcylopentadienyl)hafnium trichloride (dimethoxyethane) (0.344 g; 0.713 mmol) and toluene (4 mL) were added to a container (40 mL glass vial) and stirred. The lithium compound (0.10 g; 0.713 mmol) was added to the contents of the container in several portions and the contents of the container were stirred for 24 hours at approximately 20 °C. The contents of the container were filtered, and solvent was removed under vacuum to provide n-PrCp(4,6-dimethyl-1,2,3,4-tetrahydropentalenyl)hafnium chloride) (30% yield), the presence of additional byproducts was observed by NMR analysis. 1H NMR (400 MHz, Benzene-d6) δ 5.86 (t, J = 2.7 Hz, 2H), 5.71 (t, J = 2.7 Hz, 2H), 5.30 (s, 1H), 3.03 (ddd, J = 13.7, 8.4, 1.3 Hz, 2H), 2.77 - 2.67 (m, 2H), 2.44 (ddd, J = 14.3, 10.2, 8.0 Hz, 2H), 2.36 - 2.21 (m, 1H), 2.03 - 1.95 (m, 1H), 1.76 (s, 6H), 1.57 - 1.44 (m, 2H), 0.84 (td, J = 7.4, 5.5 Hz, 3H). The yield is reported in Table 2.

Comparative Example E was performed as follows. In a drybox, n-propylcylopentadienyl)hafnium trichloride (dimethoxyethane) (0.275 g; 0.571 mmol) and toluene (5 mL) were added to a container (40 mL glass vial) and stirred. Then, a solution of methyl magnesium bromide in diethyl ether (3.0 M; 0.50 mL; 1.49 mmol) was added dropwise to the contents of the container. Then, lithium compound (0.10 g; 0.713 mmol) was added to the contents of the container in several portions and the contents of the container were stirred for 24 hours at approximately 20 °C. Solvent was removed by vacuum and the resultant solid product was dissolved in hexanes (40 mL), stirred for 10 minutes and filtered. Hexanes were removed by vacuum to provide n-PrCp(4,6-dimethyl-1,2,3,4-tetrahydropentalenyl)hafnium dimethyl (40% yield), the presence of additional byproducts was observed by NMR analysis. 1H NMR (400 MHz, Benzene-d6) δ 5.69 (t, J = 2.7 Hz, 2H), 5.44 (t, J = 2.6 Hz, 2H), 5.07 (s, 1H), 2.55 - 2.43 (m, 6H), 2.05 - 1.96 (m, 1H), 1.89 - 1.80 (m, 1H), 1.78 (s, 6H), 1.59 (dq, J = 14.7, 7.4 Hz, 2H), 0.97 - 0.84 (m, 3H), -0.39 (s, 6H). The presence of byproduct D was identified by NMR analysis: 1H NMR (400 MHz, Benzene-d6) δ 5.73 (s, 4H), 2.24 - 2.15 (m, 2H), 1.40 (dq, J = 14.8, 7.4 Hz, 2H), 0.79 (t, J = 7.3 Hz, 3H), 0.20 (s, 9H). reported in Table 2.

**Table 2**

| | Yield |
|---|---|
| | (%) |
| Example 2 | 90 |
| Comparative Example D | 30 |
| Comparative Example E | 40 |

The data of Table 2 illustrate that Example 2 has an improved, i.e., greater, yield as compared to Comparative Examples D-E.

The data of Table 2 illustrate that Example 2 has an improved, i.e., reduced, generation of byproducts, as shown by the improved yield, as compared to Comparative Examples D-E.

Example 3, a synthesis of a bicyclic ketone compound, was performed as follows. The bicyclic ketone compound is as previously described and is made by reacting the cyclic alkene compound (as previously described; cyclopentene; R₁, R₂, and R₃ were each H) with the acid compound (as previously described; (E)-2-butenoic acid).

In a fume hood, under a nitrogen atmosphere in a vial (40 mL) equipped with a stir bar, acid compound ((E)-2-butenoic acid;1.0 g; 11.61 mmol)) was added followed by cyclic alkene compound (cyclopentene; 1.03 mL; 11.61 mmol). Next, an aprotic solvent (sulfolane, 3 mL) was added and the contents of the vial were cooled to 0 °C. Next, P₂O₅/H₃CSO₃H mixture (0.1/1) was added dropwise (5.53 mL; 34.85 mmol) at 0° C. Then the contents of the vial were warmed up to ambient temperature while stirring was maintained. At this point, visual observation was utilized to determine that the contents of the vial were homogeneous. Thereafter, the contents of the vial were stirred at ambient temperature for 24 hours. After 24 hours, the contents of the vial were slowly quenched with NaOH (3 N; 20 mL). The aqueous layer was extracted with diethyl ether (6 mL). An aliquot of the organic layer (0.25 mL ) was removed via syringe and place into a GC/MS vial, followed by dilution with diethyl ether (1.25 mL). The organic layer was characterized by GC/MS to determine product formation.

GC-MS Analysis was preformed as follows. The concentration of desired product in the crude organic product was performed using a gas chromatography system comprising an Agilent Technologies 7890A GC system equipped with an analytical column (Agilent 19091S-433 HP-5ms 0 °C - 325 °C: 15 m x 250 µm x 0.25 µm) using Helium/Hydrogen as carrier gas, coupled with a mass detector comprising an Agilent Technologies 5977B MSD analyzer operating in electronic impact (EI) mode. The raw data was analyzed by performing ion extraction of the desired product molecular weight (136 g/mol), the resulting spectra are integrated, and the area counts are reported in Table 4.

The values for relative polarity were normalized from measurements of solvent shifts of absorption spectra and were extracted from Christian Reichardt, Solvents and Solvent Effects in Organic Chemistry, Wiley-VCH Publishers, 3rd ed., 2003.

Examples 4-9 and Comparative Examples F-K were performed as Example 3, with the change that different solvents, as indicated in Table 3, were utilized.

**Table 3**

| | Solvent | Solvent type | Solvent chemistry |
|---|---|---|---|
| Example 3 | Sulfolane | Dipolar aprotic | Sulfone |
| Example 4 | Dipropylsulfone | Polar aprotic | Sulfone |
| Example 5 | 1,2-Dimethoxyethane | Polar aprotic | Glycol ether |
| Example 6 | Diethylene glycol dimethyl ether | Polar aprotic | Glycol ether |
| Example 7 | Tetraethylene glycol dimethyl ether | Polar aprotic | Glycol ether |
| Example 8 | Dichloromethane | Polar aprotic | Organo chloride |
| Example 9 | Diethyl ether | Polar aprotic | Ether |
| Comparative Example F | Dimethyl sulfoxide | Dipolar aprotic | Sulfoxide |
| Comparative Example G | Ethanol | Polar protic | Alcohol |
| Comparative Example H | tert-Butanol | Polar protic | Alcohol |
| Comparative Example I | Tetrahydrofuran | Polar aprotic | Cyclic ether |
| Comparative Example J | 1,4-Dioxane | Polar aprotic | Cyclic ether |
| Comparative Example K | Toluene | Non-polar aprotic | Aromatic hydrocarbon |

**Table 3**

| | Relative polarity | Observation during synthesis of the bicyclic ketone compound | Area count of product by GC/MS (Mass of product 1 = 136 g/mol) |
|---|---|---|---|
| Example 3 | Sulfolane | Homogeneous | 1,913,335 |
| Example 4 | Dipropylsulfone | Homogeneous | 2,177,353 |
| Example 5 | 1,2-Dimethoxyethane | Homogeneous | 2,456,533 |
| Example 6 | Diethylene glycol dimethyl ether | Homogeneous | 3,647,232 |
| Example 7 | Tetraethylene glycol dimethyl ether | Homogeneous | 3,063,840 |
| Example 8 | Dichloromethane | Homogeneous | 1,952,395 |
| Example 9 | Diethyl ether | Homogeneous | 2,856,218 |
| Comparative Example F | Dimethyl sulfoxide | Not homogeneous | 91,236 |
| Comparative Example G | Ethanol | Homogeneous | 114,755 |
| Comparative Example H | tert-Butanol | Not homogeneous | None detected |
| Comparative Example I | Tetrahydrofuran | Homogeneous¹ | 56,578 |
| Comparative Example J | 1,4-Dioxane | Not homogeneous | N/A Reaction did not proceed |
| Comparative Example K | Toluene | Not homogeneous | None detected |

Homogeneous¹. For Comparative Example I, it is noted that while homogenous, upon quenching the reaction there was precipitation and an emulsion resulted after ether extraction. This indicated an undesirable lack of solvent stability.

The data of Table 4 show that advantageously each of Examples 3-9, in contrast to each of Comparative Example F-K, each provided homogeneity when synthesizing the bicyclic ketone compound, and each provided an area count of product by GC/MS greater than 1,000,000.

## Claims

1. A method of making an alkylated metallocene having one or more tetrahydropentalenyl groups, the method comprising:
reacting a chloride compound with a lithium compound to make a reaction mixture comprising an intermediate dichloride compound; and
reacting the intermediate dichloride compound with an alkylating agent to make the alkylated metallocene having one or more tetrahydropentalenyl groups, wherein the dichloride compound is not isolated from the reaction mixture, wherein the lithium compound is represented by: 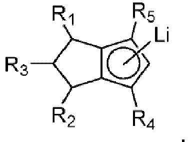
wherein R₁, R₂, R₃, R₄, and R₅ are each independently H or a (C1-C4) alkyl, and wherein the reactions occur in a same reaction vessel.

2. The method of claim 1, wherein reacting the chloride compound with the lithium compound to make the reaction mixture comprising the intermediate dichloride compound occurs in absence of the alkylating agent.

3. The method of any one of claims 1-2, wherein the chloride compound includes 3 or 4 chlorine atoms.

4. The method of any one of claims 1-3, wherein the chloride compound is selected from zirconium (IV) chloride and n-(propylcyclopentadienyl)hafniumtrichloride (dimethoxyethane).

5. The method of any one of claims 1-4, wherein the intermediate dichloride compound includes one or more tetrahydropentalenyl groups

6. The method of any one of claims 1-5, wherein the alkylating agent is an alkylmagnesium halide.

7. The method of any one of claims 1-6, wherein
the lithium compound is made by reacting butyl lithium with a bicyclic alkene compound represented by:

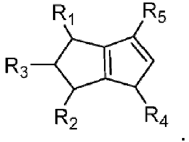

8. The method of claim 7, wherein the bicyclic alkene compound is made by reacting a bicyclic ketone compound with an alkylating agent and diethyl ether to form a bicyclic alcohol compound and dehydrating the bicyclic alcohol compound to make the bicyclic alkene compound, wherein the bicyclic ketone compound is represented by:

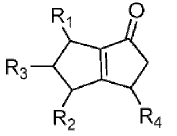

9. The method of claim 8, wherein the bicyclic ketone compound is made by reacting a cyclic alkene compound represented by: 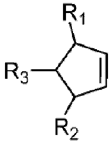 with an acid compound represented by: 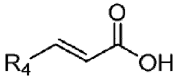 in the presence of a polar aprotic or dipolar aprotic solvent and an effective amount of a phosphoric and/or sulfonic acid reagent and under reaction conditions sufficient to make the bicyclic ketone compound.

10. The method of claim 9, wherein the solvent has a relative polarity above 0.099 and below 0.444, with the proviso that the solvent is not a cyclic ether.

11. The method of any one of claims 9-10, wherein the phosphoric and/or sulfonic acid reagent is a P₂O₅/H₃CSO₃H mixture.

## Patentansprüche

1. Verfahren zum Herstellen eines alkylierten Metallocens, das eine oder mehrere Tetrahydropentalenylgruppen aufweist, das Verfahren umfassend:
Umsetzen einer Chloridverbindung mit einer Lithiumverbindung, um ein Umsetzungsgemisch, umfassend eine intermediäre Dichloridverbindung, herzustellen; und
Umsetzen der intermediären Dichloridverbindung mit einem Alkylierungsmittel, um das alkylierte Metallocen herzustellen, das eine oder mehrere Tetrahydropentalenylgruppen aufweist, wobei die Dichloridverbindung nicht aus dem Umsetzungsgemisch isoliert wird, wobei die Lithiumverbindung dargestellt wird durch: 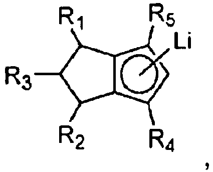
wobei R₁, R₂, R₃, R₄ und R₅ jeweils unabhängig voneinander H oder ein (C1-C4)-Alkyl sind und wobei die Umsetzungen in einem selben Umsetzungsgefäß erfolgen.

2. Verfahren nach Anspruch 1, wobei das Umsetzen der Chloridverbindung mit der Lithiumverbindung, um das Umsetzungsgemisch, umfassend die intermediäre Dichloridverbindung, herzustellen, in Abwesenheit des Alkylierungsmittels erfolgt.

3. Verfahren nach einem der Ansprüche 1 bis 2, wobei die Chloridverbindung 3 oder 4 Chloratome einschließt.

4. Verfahren nach einem der Ansprüche 1 bis 3, wobei die Chloridverbindung aus Zirconium(IV)-chlorid und n-(Propylcyclopentadienyl)hafniumtrichlorid(dimethoxyethan) ausgewählt ist.

5. Verfahren nach einem der Ansprüche 1 bis 4, wobei die intermediäre Dichloridverbindung eine oder mehrere Tetrahydropentalenylgruppen einschließt.

6. Verfahren nach einem der Ansprüche 1 bis 5, wobei das Alkylierungsmittel ein Alkylmagnesiumhalogenid ist.

7. Verfahren nach einem der Ansprüche 1 bis 6, wobei
die Lithiumverbindung durch Umsetzen von Butyllithium mit einer bizyklischen Alkenverbindung hergestellt wird, die dargestellt wird durch:

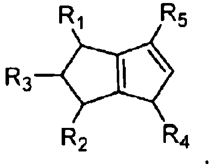

8. Verfahren nach Anspruch 7, wobei die bizyklische Alkenverbindung durch Umsetzen einer bizyklischen Ketonverbindung mit einem Alkylierungsmittel und Diethylether, um eine bizyklische Alkoholverbindung zu bilden, und Dehydratisieren der bizyklischen Alkoholverbindung, um die bizyklische Alkenverbindung herzustellen, hergestellt wird, wobei die bizyklische Ketonverbindung dargestellt wird durch:

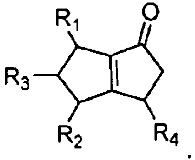

9. Verfahren nach Anspruch 8, wobei die bizyklische Ketonverbindung durch Umsetzen einer zyklischen Alkenverbindung hergestellt wird, die dargestellt wird durch: 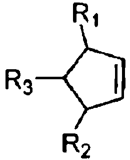
mit einer Säureverbindung, die dargestellt wird durch: 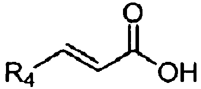
in Gegenwart eines polaren aprotischen oder dipolaren aprotischen Lösungsmittels und einer wirksamen Menge eines Phosphor- und/oder Sulfonsäurereagens und unter Umsetzungsbedingungen, die ausreichen, um die bizyklische Ketonverbindung herzustellen.

10. Verfahren nach Anspruch 9, wobei das Lösungsmittel eine relative Polarität über 0,099 und unter 0,444 aufweist, mit der Maßgabe, dass das Lösungsmittel kein zyklischer Ether ist.

11. Verfahren nach einem der Ansprüche 9 bis 10, wobei das Phosphor- und/oder Sulfonsäurereagens ein P₂O₅/H₅CSO₃H-Gemisch ist.

## Revendications

1. Procédé de préparation d'un métallocène alkylé ayant un ou plusieurs groupes tétrahydropentalényle, le procédé comprenant :
la réaction d'un composé chlorure avec un composé lithium pour préparer un mélange réactionnel comprenant un composé dichlorure intermédiaire ; et
la réaction du composé dichlorure intermédiaire avec un agent alkylant pour préparer le métallocène alkylé ayant un ou plusieurs groupes tétrahydropentalényle, dans lequel le composé dichlorure n'est pas isolé du mélange réactionnel, dans lequel le composé lithium est représenté par : 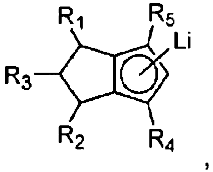
où R₁, R₂, R₃, R₄, et R₅ sont chacun indépendamment H ou un alkyle en C1-C4, et dans lequel la réaction se produit dans une même cuve de réaction.

2. Procédé selon la revendication 1, dans lequel la réaction du composé chlorure avec le composé lithium pour préparer le mélange réactionnel comprenant le composé dichlorure intermédiaire se produit en l'absence de l'agent alkylant.

3. Procédé selon l'une quelconque des revendications 1 à 2, dans lequel le composé chlorure comporte 3 à 4 atomes de chlore.

4. Procédé selon l'une quelconque des revendications 1 à 3, dans lequel le composé chlorure est choisi parmi chlorure de zirconium (IV) et trichlorure de n-(propylcyclopentadiényl)hafnium (diméthoxyéthane).

5. Procédé selon l'une quelconque des revendications 1 à 4, dans lequel le composé dichlorure intermédiaire comporte un ou plusieurs groupes tétrahydropentalényle

6. Procédé selon l'une quelconque des revendications 1 à 5, dans lequel l'agent alkylant est un halogénure d'alkylmagnésium.

7. Procédé selon l'une quelconque des revendications 1 à 6, dans lequel
le composé lithium est préparé en faisant réagir du butyllithium avec un composé alcène bicyclique représenté par :

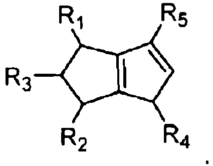

8. Procédé selon la revendication 7, dans lequel le composé alcène bicyclique est préparé en faisant réagir un composé cétone bicyclique avec un agent alkylant et de l'éther diéthylique pour former un composé alcool bicyclique et en déshydratant le composé alcool bicyclique pour préparer le composé alcène bicyclique, dans lequel le composé cétone bicyclique est représenté par :

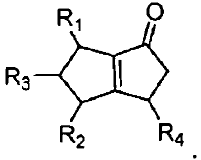

9. Procédé selon la revendication 8, dans lequel le composé cétone bicyclique est préparé en faisant réagir un composé alcène cyclique représenté par : 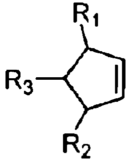 avec un composé acide représenté par : 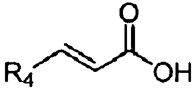 en présence d'un solvant aprotique polaire ou aprotique dipolaire et d'une quantité efficace d'un réactif acide phosphorique et/ou sulfonique et dans des conditions de réaction suffisantes pour préparer le composé cétone bicyclique.

10. Procédé selon la revendication 9, dans lequel le solvant a une polarité relative supérieure à 0,099 et inférieure à 0,444, à condition que le solvant ne soit pas un éther cyclique.

11. Procédé selon l'une quelconque des revendications 9 à 10, dans lequel le réactif acide phosphorique et/ou sulfonique est un mélange P₂O₅/H₃CSO₃H.
